Europäisches Patentamt

European Patent Office

Office européen des brevets

(19)

(11) Numéro de publication: **0 004 243**
**B1**

(12) **FASCICULE DE BREVET EUROPEEN**

(45) Date de publication du fascicule du brevet: **08.08.84**

(51) Int. Cl.³: **A 61 N 1/36, A 61 N 1/08**

(21) Numéro de dépôt: **79400156.0**

(22) Date de dépôt: **09.03.79**

(54) Stimulateur cardiaque implantable à fonctions thérapeutique et diagnostique.

(30) Priorité: **14.03.78 FR 7807270**

(43) Date de publication de la demande:
**19.09.79 Bulletin 79/19**

(45) Mention de la délivrance du brevet:
**08.08.84 Bulletin 84/32**

(84) Etats contractants désignés:
**BE CH DE GB IT LU NL SE**

(56) Documents cités:
**FR - A - 2 147 129
FR - A - 2 166 517
FR - A - 2 167 560
FR - A - 2 311 528
FR - A - 2 366 012
GB - A - 2 006 015
US - A - 3 688 092
US - A - 3 920 005
US - A - 4 050 004**

(73) Titulaire: **CARDIOFRANCE - COMPAGNIE FRANCAISE D'ELECTROCARDIOLOGIE
73, route de Neuilly
F-93160 Noisy Le Grand (FR)**

(72) Inventeur: **Buffet, Jacques
28, avenue Thiers
F-93340 Le Raincy (FR)**

(74) Mandataire: **Derambure, Christian
Cabinet BUGNION ASSOCIES SARL 116,
boulevard Haussmann
F-75008 Paris (FR)**

Courier Press, Leamington Spa, England.

## Description

L'invention concerne un stimulateur cardiaque de type sentinelle pour stimuler automatiquement les insuffisances cardiaques du patient.

Aujourd'hui, il est courant d'implanter chez un patient présentant des insuffisances cardiaques qui ont pu être préalablement diagnostiquées, des stimulateurs remplissant une fonction thérapeutique en vue précisément de pallier ces insuffisances cardiaques. Dans d'autres cas, le diagnostic ne permet pas de mettre en évidence de façon suffisamment certaine l'origine cardiologique des troubles dont souffre le patient. Dans ces hypothèses, il y a tout autant de risque de ne pas implanter de stimulateur que d'en implanter un. De plus, il n'est guère possible, a posteriori, de contrôler l'état et le degré de dépendance du patient par rapport au stimulateur cardiaque donc l'utilité de celui-ci.

On connaît déjà, pour résoudre ce problème, des appareils enregistreurs en totalité extra-corporels et d'ailleurs volumineux. Ceux-ci sont gênants et ne donnent pas, le plus souvent, des résultats fiables.

On connaît par ailleurs des stimulateurs appelés sentinelles, dont le circuit de stimulation est bloqué pendant un temps correspondant à la période de base du stimulateur. Si le coeur ne s'est pas manifesté pendant cette période, l'impulsion se déclenche. Si le coeur s'est manifesté pendant la période de base, le circuit est inhibé et ne pourra délivrer une impulsion qu'après l'écoulement d'une autre période de base. On a donc un stimulateur "à la demande".

Cependant, de tels stimulateurs peuvent comporter des défauts de fonctionnement, à savoir d'une part une perte de stimulation, c'est-à-dire que le stimulateur n'envoie pas d'impulsions de stimulation correctes par suite du déplacement de l'électrode ou par suite de pannes électroniques, et d'autre part un défaut de détection d'une activité cardiaque propre, ce qui conduit à envoyer des impulsions de stimulation, alors que ce n'est pas nécessaire et donc à provoquer une fibrillation du coeur.

Pour détecter ces défauts de fonctionnement, on a décrit dans le brevet français No. 2 311 528 un stimulateur sentinelle comportant un boîtier et dans celui-ci des moyens de génération d'impulsions de stimulation à des intervalles de temps T, des moyens de contrôle des impulsions de stimulation et des moyens de détection d'une activité cardiaque pendant un intervalle de temps t inférieure à T.

De tels stimulateurs ne permettent de mettre en évidence l'origine cardiologique des troubles dont souffre le patient.

L'invention vise à remédier à ces inconvénients et à cet effet, elle propose un stimulateur cardiaque de type sentinelle comprenant deus électrodes destinées à être reliées à une sonde cardiaque; une source d'énergie électrique autonome; un amplificateur relié en entrée aux électrodes et un filtre relié en entrée à l'amplificateur pour respectivement amplifier et filtrer les signaux d'électrode en provenance de la sonde cardiaque; un détecteur de niveau préfixé relié en entrée au filtre pour détecter une activité cardiaque autonome du patient, et un étage de sortie du stimulateur pour appliquer des impulsions de stimulation cardiaque à une des deux électrodes, caractérisé par le fait qu'il comporte entre le détecteur de niveau et l'étage de sortie un module logique relié aux connexions de la source d'énergie électrique autonome et également relié à une relais magnétique pour contrôler l'activité cardiaque détectée par le détecteur de niveau et stimuler automatiquement les insuffisances cardiaques en appliquant des impulsions isolées et successives à la première électrode par l'étage de sortie et, à partir d'une commande émise par le relais, pour contrôler a posteriori l'activité cardiaque stimulée par le stimulateur, le module logique comprenant une unité centrale pour l'exécution d'un programme de stimulation automatique ou d'un programme de contrôle à postériori en fonction de l'activité cardiaque détecté; une mémoire reliée à l'unité centrale par un circuit de connexion "bus" et comprenant deux registres pour respectivement enregistrer le nombre d'impulsions de stimulation successives et le nombre d'impulsions de stimulation isolées appliquées à la première électrode et enregistrer le type des impulsions successives ou isolées enregistrées dans le premier registre; une horloge; un circuit doubleur de tension relié en entrée à l'horloge et en sortie à l'unité centrale et à la mémoire; une porte logique "OU" reliée par sa sortie à une entrée "interruption" de l'unité centrale et par deux entrées au détecteur de niveau et au relais respectivement par l'intermédiaire de deux connexions pour interrompre l'exécution d'un programme dans l'unité centrale par le détecteur de niveau dans le fonctionnement de stimulation automatique ou par le relais dans le fonctionnement de contrôle a posteriori; l'unité centrale comprenant en outre deux entrées "indicateurs" reliées au détecteur de niveau et au relais respectivement par les deux connexions pour indiquer à l'unité centrale le type d'interruption soit par le détecteur de niveau dans le fonctionnement de stimulation automatique, soit par le relais dans le fonctionnement de contrôle a posteriori; le relais étant relié dans un état déterminé à la porte logique OU pour commander la lecture du contenu des deux registres.

Les autres caractéristiques de l'invention résulteront de la description qui suit en référence aux dessins annexés dans lesquels:

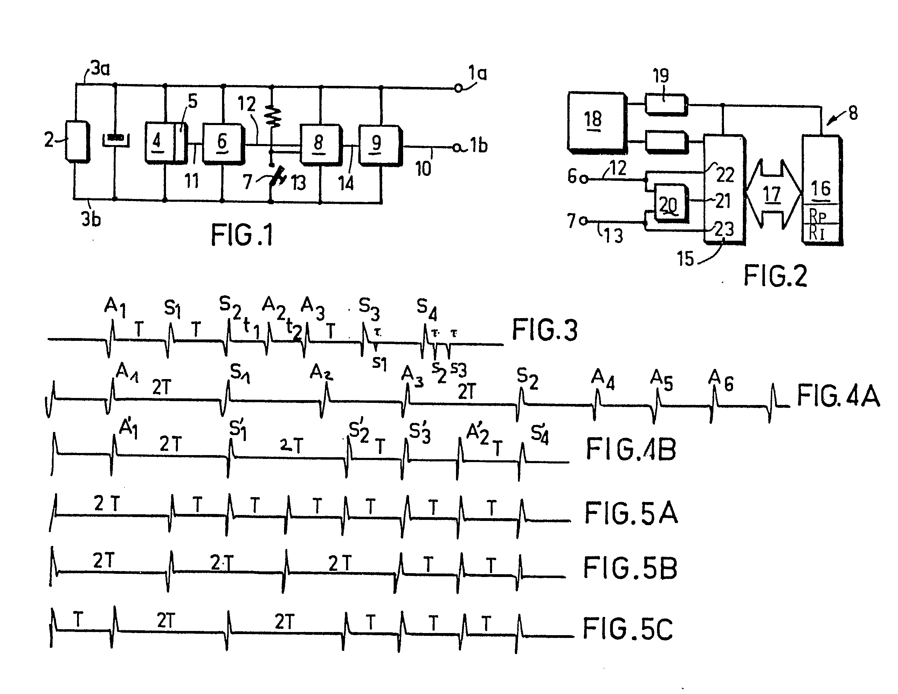

La figure 1 est une vue schématique illustrant un stimulateur cardiaque suivant l'invention; la figure 2 est une vue schématique du module logique faisant partie du stimulateur cardiaque; la figure 3 est une vue schématique illustrant le fonctionnement du stimulateur suivant le programme général; la figure 4A est un schéma illustrant un déroulement possible des phases d'un programme de contrôle; la figure 4B est un schéma illustrant un autre déroulement possible d'un programme de contrôle; les figures 5A, 5B, 5C sont trois schémas illustrant les enregistrements obtenus grâce au stimulateur suivant l'invention.

Suivant l'invention, il est proposé un stimulateur cardiaque implantable, destiné à être associé à une sonde cardiaque non représentée, comprenant un boîtier, non représenté, et dans celui-ci des moyens assurant automatiquement la fonction thérapeutique normale du stimulateur en vue de pallier les insuffisances cardiaques du patient. Le stimulateur comporte en outre, à l'intérieur même du boitier, des moyens assurant une fonction de contrôle a posteriori de l'état et du degré de dépendance du patient par rapport au stimulateur cardiaque assurant sa fonction thérapeutique. Des moyens de commande externes assurent la commande des moyens remplissant la fonction de contrôle.

Les moyens remplissant les fonctions thérapeutiques et de contrôle comprennent: deux électrodes 1*a*, 1*b*, destinées à être reliées à la sonde cardiaque par des moyens appropriés; une source d'énergie électrique autonome 2 telle qu'une pile pourvue des connexions 3*a* et 3*b* dont l'une, soit 3*a*, par exemple est reliée à l'électrode 1*a*. Entre les connexions 3*a* et 3*b*, sont placés en parallèle les éléments suivants: un amplificateur 4 et son filtre 5; un détecteur de niveau 6; un relais magnétique 7; une module logique 8; et un étage de sortie 9 relié par une connexion 10 à l'électrode 1*b*. Des connexions 11, 12, 13, 14 relient respectivement l'amplificateur 4 et son filtre 5 au détecteur de niveau 6, ce dernier au module logique 8, le relais magnétique 7 également au module logique 8 et celui-ci à l'étage de sortie 9.

L'amplificateur 4 et son filtre 5 ont comme fonction d'amplifier et de filtrer les signaux qui sont détectés sur les électrodes 1*a*, 1*b*. Son coefficient d'amplification est notamment de l'ordre de 500 en tension environ. Le filtre 5 est notamment du type basse-bande centré sur une fréquence égale ou voisine de 60 hertz.

Le détecteur de niveau 6 opère une commutation lorsque le niveau en sortie de l'amplificateur 4 et du filtre 5 dépasse une valeur préfixée par exemple de 0,5 volts environ. Il résulte de ce dispositif que tout signal détecté sur les électrodes, accordé à la bande passante et dont l'amplitude une fois amplifié, dépassera le seuil de commutation du détecteur 6, entraîne la commutation de celui-ci.

Le module logique 8, qui sera décrit plus en détail ci-après a comme fonction d'élaborer des impulsions de stimulation en synchronisme avec les signaux en sortie du détecteur de niveau 6.

L'étage de sortie 9 a comme fonction de délivrer à l'électrode 1*b*, des impulsions calibrées et mise en forme selon les signaux en sortie du module logique 8.

Le relais magnétique 7 se présentant notamment sous la forme d'un interrupteur à lame souple est normalement en position de circuit ouvert. Ce relais est susceptible d'être basculé en position de circuit fermé par des moyens de commande amovibles externes, notamment des moyens magnétiques, et en particulier un organe produisant un champ magnétique tel qu'un aimant permanent.

Le module logique 8 comprend une unité centrale 15 pour l'exécution de programmes et d'opérations d'enregistrement et de lecture qui seront décrits dans la suite, une mémoire 16 pour mémoriser les programmes à exécuter, reliée à l'unité centrale 15 par un circuit de connexion "bus" 17 et comprenant deux registres Rp et Ri respectivement pour enregistrer le nombre des impulsions successives et le nombre des impulsions isolées appliquées par l'étage de sortie 9 à l'électrode 1*b* et pour enregistrer le type des impulsions successives ou isolées enregistrées dans le registre Rp, une horloge 18 pour engendrer des impulsions de base de fonctionnement de l'unité centrale et de la mémoire, un circuit doubleur de tension 19 relié en entrée à l'horloge 18 et en sortie à l'unité centrale 15 et à la mémoire 16, une porte logique OU 20 dont la sortie est reliée à une entrée "interruption" 21 de l'unité centrale pour commander une interruption d'exécution de programme et dont deux entrées sont respectivement reliées par les connexions 12 et 13 au détecteur de niveau 6 et au relais 7. L'unité centrale 15 comporte en outre deux entrées "indicateurs" 22, 23, respectivement reliées par les connexions 12 et 13 au détecteur de niveau 6 et au relais 7 pour indiquer à l'unité centrale le type d'interruption, soit par le détecteur de niveau 6 dans un fonctionnement de stimulation cardiaque automatique, soit par l'interrupteur 7 dans un fonctionnement de contrôle a posteriori. L'unité centrale 15 est aussi reliée à l'étage de sortie 9 par une sortie 14 du module logique 8 pour permettre à l'étage de sortie d'appliquer à l'électrode 1*b* les impulsions de stimulation successives et isolées engendrées par l'exécution d'un programme dans l'unité centrale.

Tous ces circuits sont préférentiellement réalisés selon la technologie à transistor MOS. Cela permet notamment un fonctionnement à des tensions d'alimentation très faibles et une consommation de courant peu élevée à basse fréquence.

L'horloge 18 est réalisée de toute façon appropriée. En particulier, il s'agit d'un oscillateur réalisé à partir d'inverseurs logiques et d'un circuit à résistance-capacité. Elle est alimentée par la pile par l'intermédiaire d'un condensateur de découplage. Sa fréquence d'oscillation dépend du temps de charge du condensateur jusqu'au seuil de la porte logique.

Le doubleur de tension est réalisé à l'aide de deux condensateurs disposés alternativement en parallèle et en série à la fréquence d'oscillation de l'horloge. On obtient ainsi une tension d'alimentation suffisante pour le module logique même lorsque la tension de la pile d'alimentation de l'horloge diminue.

L'unité centrale par microprocesseur fonctionne de manière classique à partir d'une logique de contrôle, d'une unité de calcul arithmétique et logique, de registres de programmes et de données et d'un registre accumulateur. Elle réalise un programme dont les instructions sont inscrites dans la mémoire, dont le déroulement est commandé par l'horloge 18.

Comme on l'a déjà signalé, le stimulateur, tel qu'il vient d'être décrit dans ses éléments fonctionnels, peut remplir sa fonction thérapeutique normale et habituelle ce qui correspond à un programme générale et, également, une fonction de contrôle correspondant à un programme particulier.

Les caractéristiques du programme général sont les suivantes:

En l'absence d'activité cardiaque autonome détectée sur les électrodes, l'appareil émet des impulsions de stimulation à une période régulière T connue et déterminée, notamment comprise entre 500 et 1.300 millisecondes par exemple.

Toute détection d'une activité cardiaque autonome sur les électrodes, provoque le recyclage simultané de l'appareil pour une nouvelle période T.

Après chaque impulsion de stimulation, ou chaque onde correspondant à une activité cardiaque autonome, court une période dite "réfractaire" telle que tout onde détectée pendant cette période ne provoque par le recyclage du stimulateur. Si cependant une première onde est détectée pendant cette période, la période réfractaire est prolongée d'une seconde période réfractaire. Cette disposition est telle que les parasites électromagnétiques ayant une période de répétition inférieure à la période réfractaire ne provoquent pas le recyclage du stimulateur.

Si le relais magnétique 7 est mis en court-circuit par action des moyens de commande externes, le recyclage est rendu inopérant et le stimulateur émet en permanence des impulsions avec la période T.

Sur la figure 3, est illustré le fonctionnement du stimulateur suivant le programme général.

En A1 est représentée une onde autonome. Aucune activité cardiaque autonome ne faisant suite à cette onde A1, le stimulateur émet, à l'issue de la période T, une impulsion de stimulation S1. De même, aucune onde autonome n'apparaissant le stimulateur émet, à l'issue de la période T après S1, une impulsion de stimulation S2. Si, à l'issue d'un temps t1 inférieur à T, apparaît une onde cardiaque autonome A2, le stimulateur cardiaque est recyclé à partir de ce moment. Si après l'onde A2, apparaît une onde cardiaque autonome A3 à l'issue d'un temps t2 inférieur à T, le stimulateur est encore recyclé en A3. Si donc, après A3, apparaît une pause cardiaque au moins égale à T, le stimulateur émet une impulsion de stimulation S3, à l'issue de la période T après A3. L'impulsion S3 fait courir la période réfractaire de durée $\tau$. On observe que l'onde s1 intervenant après S3, dans la période réfractaire $\tau$, ne provoque pas le recyclage du stimulateur de sorte qu'une impulsion de stimulation apparaît après S3, à l'issue de la période T. De même, l'impulsion S3 fait courir une période réfractaire de durée $\tau$ pendant laquelle apparaît une onde s2 qui d'une part, ne provoque pas le recyclage du stimulateur et, d'autre part, fait courir une deuxième période réfractaire de même durée $\tau$ pendant laquelle peut apparaître une onde s3 qui comme s2 ne provoque pas le recyclage du stimulateur.

Le programme particulier présente les caractéristiques suivantes:

— La première impulsion de stimulation est délivrée après une pause cardiaque due à une absence d'activité cardiaque autonome pendant une durée 2T égale au double de la période de stimulation normale (Par exemple, si la période T est égale à 1.000 millisecondes, le stimulateur attendra 2.000 millisecondes avant que de stimuler.

— Après cette première impulsion de stimulation, il est émis en l'absence de toute activité cardiaque autonome, une deuxième impulsion de stimulation après une durée 2T.

— Lorsqu'une première et une deuxième impulsions successives telles que celles qui viennent d'être décrites ont été délivrées, le stimulateur émet des impulsions successives à une période T.

— Tant qu'une première et une deuxième impulsions successives n'ont pas été émises, comme indiqué précédemment, le stimulateur fonctionne suivant le programme particulier décrit. Au contraire, dès que le stimulateur a émis la première et la deuxième impulsions successives, comme décrit, il fonctionne non pas suivant le programme particulier mais suivant le programme général déjà décrit.

Les figures 4A et 4B illustrent deux déroulements possibles du programme particulier.

Sur la figure 4A, on trouve en A1 une onde autonome. En l'absence d'activité cardiaque, la première impulsion de stimulation S1 apparaît à l'issue d'une durée 2T après A1. En A2 apparaît une onde cardiaque autonome à l'issue d'une durée inférieure à 2T après S1, de même en ce qui concerne l'onde autonome A3 successive à A2. Etant donné l'apparition des ondes A2 et A3, le stimulateur est recyclé et l'impulsion de stimulation S2 apparaît en cas de pause cardiaque au moins égale à 2T. Les ondes autonomes successives A4, A5 et A6 suivent l'impulsion de stimulation S2 et provoquent donc le recyclage du stimulateur. Dans cette hypothèse, les impulsions de stimulation S1 et S2 constituent deux premières impulsions isolées en ce sens que l'impulsion S2 ne constitue pas une deuxième impulsion successive à l'impulsion S1.

Sur la figure 4B, est représenté un autre fonctionnement possible dans lequel on trouve A'1 une onde cardiaque autonome, en S'1 une impulsion de stimulation survenant après une durée 2T à compter de A'1. Si, après l'impulsion S'1, aucune activité cardiaque autonome n'apparaît (contrairement à ce que l'on a vu précédemment en A2 et A3 en référence à la figure 4A) et ce, pendant une durée égale à 2T, le stimulateur émet une deuxième impulsion de stimulation S'2 successive à S'1 et écartée de celle-ci dans le temps de 2T. Si, à l'issue de l'impulsion S'2, aucune activité cardiaque autonome n'apparait et ce pendant une durée égale à T, le stimulateur émet en S'3 une impulsion de stimulation à l'issue de la période T après S'2. Ace moment, le stimulateur suit le programme général de sorte qu'il émet normalement des impulsions à la période T, en l'absence de toute activité cardiaque autonome. Par exemple, il est illustré en A'2, une onde cardiaque autonome intervenant après S'3, à l'issue d'une durée inférieure à T. Le stimulateur se recycle donc à partir de A'2 et émet par exemple en S'4, après une durée T après A'2, une impulsion de stimulation. Dans ce cas de figure, le stimulateur a émis en S'1, S'2, et S'3, trois coups successifs selon le programme particulier. Comme on l'a déjà mentionné, dans le cas de la figure 4A, l'appareil n'ayant pas émis d'impulsions successives selon le programme particulier, continue d'appliquer ce dernier. Par contre, dans le cas de la figure 4B, le stimulateur cesse de fonctionner suivant le programme particulier et fonctionne suivant le programme général.

Le nombre d'impulsions de stimulation délivrées est enregistré dans le registre Rp de la mémoire 16. Plus précisément, sont enregistrés dans le registre Rp:

Soit le nombre d'impulsions de stimulation délivrées si le stimulateur n'a jamais délivré au moins deux impulsions successives soit une première et une deuxième impulsions selon le programme particulier. Ce cas correspond à l'hypothèse de la figure 4A. Dans ce cas, il est enregistré les deux impulsions S1 et S2. Ces deux impulsions correspondent aux deux fois où le patient a présenté une pause ventriculaire d'une durée supérieure à deux fois et inférieure à quatre fois la période de stimulation. En l'occurrence, l'impulsion S1 correspond à la pause ventriculaire A1, A2 et l'impulsion de stimulation S2 à la pause ventriculaire A3, A4 qui toutes deux ont effectivement une durée au moins égale à 2T et inférieure à 4T.

Soit le nombre d'impulsions de stimulation successives délivrées. Cette hypothèse correspond à la figure 4B où il est émis les trois impulsions successives S'1, S'2 et S'3. Ce nombre caractérise la durée de la première pause ventriculaire supérieure à 4T. En l'occurrence, il s'agit de la pause ventriculaire A'1, A'2 qui, dans le cas de la figure 4B, a une durée comprise entre 5T et 6T.

Les valeurs inscrites dans le registre Rp sont les suivantes: une première impulsion de stimulation isolée inscrira 1. Une deuxième impulsion de stimulation isolée inscrira 2. Une troisième impulsion de stimulation isolée inscrira 3 et ainsi de suite en ce qui concerne les impulsions de stimulation isolées. Si, par exemple, après la troisième impulsion de stimulation isolée, l'appareil émet une impulsion de stimulation successive, il sera inscrit dans le registre non pas 4 mais 2. Autrement dit, le contenu du registre Rp est remis à 2, dès qu'une deuxième impulsion successive est délivrée. Si après cette impulsion, l'appareil émet d'autres impulsions successives, celles-ci, seront respectivement notées 3, 4, etc.

L'enregistrement cesse dans l'une des deux hypothèses suivantes:

Apparition d'une activité cardiaque autonome après au moins deux impulsions de stimulation successives délivrées.

Remplissage de la capacité du registre Rp.

Le registre Ri est en correspondance avec le registre Rp et indique si les impulsions de stimulation dont le nombre est inscrit dans Rp sont de type isolé (cas de la figure 4a si le stimulateur n'a pas délivré deux impulsions successives) ou successives (cas de la figure 4B).

La lecture des enregistrements est réalisée de la manière suivante:

Grâce aux moyens de commande amovibles externes tels qu'un aiment permanent, on bascule le relais magnétique 7 en position de circuit fermé.

On mesure ensuite la succession de périodes de stimulation obtenues.

Dans le cas où aucun enregistrement n'est intervenu, on obtient une représentation telle que celle illustrée sur la figure 5A où la deuxième onde est séparée de la première par 2T et les ondes successives séparées l'une de l'autre de la période T.

Dans l'hypothèse où le stimulateur a enregistré plusieurs impulsions de stimulation isolées, on obtient une représentation telle que la figure 5B qui correspond à l'enregistrement de deux impulsions de stimulation, dans ce cas, on obtient les intervalles de temps successifs suivants: 2T, 2T, 2T, T, T, T, etc.

Dans le cas où l'on a enregistré plusieurs impulsions de stimulation successives, on obtient un enregistrement du type de celui représenté sur la figure 5C pour deux impulsions successives. La succession des impulsions se fait alors suivant les intervalles de temps T, 2T, 2T, T, T, etc.

Il est donc clair que le seul examen des enregistrements permet, en mesurant les intervalles entre les impulsions, de déterminer si celles-ci correspondent à des impulsions successives ou à des impulsions isolées. On peut donc en déduire, comme on l'a vu précédemment, d'une part, le nombre de fois où le patient a présenté une pause cardiaque d'une durée supérieure à deux fois et inférieure à quatre fois la période de stimulation et, d'autre part, la longueur de la première pause cardiaque d'une durée supérieure à quatre fois la période de stimulation. Ces éléments permettent donc de contrôler l'état et le degré de dépendance du patient par rapport à son stimulateur cardiaque.

Il est prévu, en outre, des moyens d'effacement des enregistrements et de débrayage du mécanisme d'enregistrement commandés de façon externe.

## Revendications

1. Stimulateur cardiaque de type sentinelle comprenant deux électrodes destinées à être reliées à une sonde cardiaque; une source d'énergie électrique autonome; un amplificateur relié en entrée aux électrodes et un filtre relié en entrée à l'amplificateur pour respectivement amplifier et filtrer les signaux d'électrodes en provenance de la sonde cardiaque; un détecteur de niveau préfixé relié en entrée au filtre pour détecter une activité cardiaque autonome du patient, et un étage de sortie du stimulateur pour appliquer des impulsions de stimulation cardiaque à une des deux électrodes, caractérisé par le fait qu'il comporte entre le détecteur de niveau (6) et l'étage de sortie (9), un module logique (8) relié aux connexions (3a, 3b) de la source d'énergie électrique autonome (2) et également relié à un relais magnétique (7), pour contrôler l'activité cardiaque détectée par le détecteur de niveau (6) et stimuler automatiquement les insuffisances cardiaques en appliquant des impulsions isolées et successives à la première électrode (1b) par l'étage de sortie (9) et, à partir d'une commande émise par le relais (7) pour contrôler a posteriori l'activité cardiaque stimulée par le stimulateur, le module logique (8) comprenant une unité centrale (15) pour l'exécution d'un programme de stimulation automatique ou d'un programme de contrôle à postériori, en fonction de l'activité cardiaque détectée; une mémoire (16) reliée à l'unité centrale (15) par un circuit de connexion "bus" (17) et comprenant deux registres (Rp, Ri) pour respectivement enregistrer le nombre d'impulsions de stimulation successives et le nombre d'impulsions de stimulation isolées appliquées à la première électrode (1b) et enregistrer le type des impulsions successives ou isolées enregistrées dans le premier registre (Rp); une horloge (18); un circuit doubleur de tension (19) relié en entrée à l'horloge (18) et en sortie à l'unité centrale (15) et à la mémoire (16); une porte logique OU (20) reliée par sa sortie à une entrée "interruption" (21) de l'unité centrale (15) et par deux entrées au détecteur de niveau (6) et au relais (7) respectivement par l'intermédiaire de deux connexions (12, 13) pour interrompre l'exécution d'un programme dans l'unité centrale par le détecteur de niveau dans le fonctionnement de stimulation automatique ou par le relais dans le fonctionnement de contrôle a posteriori; l'unité centrale comprenant en outre deux entrées "indicateurs" (21, 23) reliées au détecteur de niveau (6) et au relais (7) respectivement par les deux connexions (12, 13) pour indiquer à l'unité centrale le type d'interruption soit par le détecteur de niveau dans le fonctionnement de stimulation automatique, soit par le relais dans le fonctionnement de contrôle a posteriori; le relais (7) étant relié dans un état déterminé à la porte logique OU (20) pour commander la lecture du contenu des deux registres (Rp, Ri).

2. Stimulateur cardiaque suivant la revendication 1, caractérisé par le fait que l'horloge (18) est réalisée au moyen d'un oscillateur constitué à partir d'inverseurs logiques et un circuit à résistance-capacité, alimentés par la source d'énergie par l'intermédiaire d'un condensateur de découplage.

## Patentansprüche

1. Implantierbarer Herzschrittmacher mit einer therapeutischen und einer diagnostischen Funktion bestehend aus zwei Elektroden zum Anschließen an einen Herzmeßfühler, aus einer unabhängigen elektrischen Stromquelle, einem am Eingang der Elektroden liegenden Verstärker und einem am Eingang des Verstärkers liegenden Filter zum Verstärken bzw. Filtern der vom Herzmeßfühler kommenden Elektrosignale, einem Detektor mit voreingestelltem Pegel, der zum Erfassen einer selbständigen Herztätigkeit des Patienten am Eingang des Filters liegt, und aus einer Ausgangsstufe des Schrittmachers, um Herzstimulationsimpulse an eine der beiden Elektroden zu geben, dadurch gekennzeichnet, daß zwischen dem Pegeldetektor (6) und der Ausgangsstufe (9) ein Logikmodul liegt, der an den Anschlußstellen (3a, 3b) der unabhängigen elektrischen Stromquelle (2) und darüber hinaus an einem Magnetrelais (7) liegt, um die vom Pegeldetektor (8) erfasste Herztätigkeit zu steuern und selbsttätig auf mangelnde Herztätigkeit (Insuffizienz) stimulierend einzuwirken, indem vom Stufenausgang (9) isolierte und sukzessive Impulse an die erste Elektrode gegeben werden, und um ausgehend von einem vom Relais (7) ausgesandten Steuerimpuls die vom Herzschrittmacher angereizte Herztätigkeit a posteriori zu kontrollieren, und daß das Logikmodul (8) eine Zentraleinheit (15) zum Durchführen eines Programms der selbsttätigen Stimulierung oder eines Programms der Kontrolle a posteriori in Abhängigkeit von der erfassten Herztätigkeit aufweist, sowie gekennzeichnet durch eine über eine "Bus"-Anschluß- schaltung (17) an der Zentraleinheit (15) liegende Speichereinheit (16), die zwei Register (Rp, Ri) aufweist, um die Anzahl der sukzessiven Stimulationsimpulse bzw. die an die erste Elektrode (1b) gegebene Anzahl von isolierten Stimulationsimpulsen sowie die Art der im ersten Register (Rp) aufgezeichneten sukzessiven oder isolierten Impulse aufzuzeichnen, durch einen Taktgeber (18), durch einen am Eingang des Taktgebers (18) und am Ausgang der Zentraleinheit (15) und der Speichereinheit (16) liegende Verdopplungsschaltung, durch ein logisches ODER-Tor (20), das über zwei Anschlüsse (12, 13) mit einem Ausgang am Eingang "Unterbrechung" (21) der Zentraleinheit (15) und mit zwei Eingängen am Pegeldetektor (6) bzw. am Relais (7) liegt, um die Durchführung eines Programms in der Zentraleinheit durch den Pegeldetektor beim selbst- tätigen Stimulationsbetrieb oder den Kontroll- betrieb a posteriori durch das Relais zu unterbrechen, wobei die Zentraleinheit darüber hinaus zwei "Anzeiger"-Eingänge (21, 23) besitzt, die über die beiden Anschlüsse (12, 13) am Pegeldetektor (6) bzw. am Relais (7) liegen, um der Zentraleinheit die Art der Unterbrechung entweder durch den Pegeldetektor beim selbsttätigen Stimulationsbetrieb oder durch das Relais beim Kontrollbetrieb a posteriori anzuzeigen, und wobei das Relais 7 in einem festgelegten, d.h. determinierten Zustand am logischen ODER-Tor (20) liegt, um das Lesen des Inhalts beider Register (Rp, Ri) zu steuern.

2. Herzschrittmacher nach Anspruch 1, dadurch gekennzeichnet, daß der Taktgeber unter Zuhilfenahme eines Oszillators gebildet ist, der von logischen NICHT-Gliedern ausgehend ein RC-Glied umfasst, die über einen Kopplungskondensator von der Energiequelle gespeist werden.

**Claims**

1. Cardiac pacemaker of sentinel type comprising two electrodes intended for connection to a cardiac probe; a source of autonomous electrical energy; an amplifier connected to the input of the electrodes and a filter connected to the input of the amplifier in order respectively to amplify and filter the electrode signals emanating from the cardiac probe; a pre- attached level detector connected to the input of the filter to detect autonomous cardiac activity of the patient, and a pacemaker output stage to apply cardiac stimulation impulses to one of the two electrodes, characterized by the fact that it includes, between the level detector (6) and the output stage (9), a logic module (8) linked to the connections (3a, 3b) of the auto- monous electrical energy source (2) and likewise linked to a magnetic relay (7) to monitor cardiac activity detected by the level detector (6) and automatically stimulate cardiac insufficiencies by delivering isolated and successive impulses to the first electrode (1b) by the output stage (9) and, following an actuation from the relay (7) to monitor a posteriori cardiac activity stimulated by the pacemaker, the logic module (8) comprising a central unit (15) for executing an automatic stimulation programme or an posteriori monitoring programme, in relation to the cardiac activity detected; a memory (16) linked to the central unit (15) by a "bus" connection circuit (17) and comprising two registers (Rp, Ri) to register respectively the number of successive stimulation impulses and the number of isolated stimulation impulses delivered to the first electrode (1b) and to register the type of successive or isolated impulses registered in the first register (Rp); a clock (18); a doubler voltage circuit (19) linked to the input side of the clock (18) and to the output side of the central unit (15) and of the memory (16); a logic gate OU (20) linked on its output side to an interrupter lead-in (21) on the central unit (15) and at two lead-ins to the level detector (6) and to the relay (7) respectively through the intermediary of two connections (12, 13) to interrupt execution of a programme in the central unit by the level detector in the operation of automatic stimulation or by the relay in the operation of a posteriori monitoring; the central unit including moreover two lead-in "indicators" (21, 23) linked to the level indicator (6) and to the relay (7) respectively by the two connections (12, 13) to indicate to the central unit the type of interruption either by the level detector in the operation of automatic stimulation, or by the relay in the operation of a posteriori monitoring; the relay (7) being linked in a given state to the logic gate OU (20) to actuate reading of the content of the two registers (Rp, Ri).

2. Cardiac pacemaker in accordance with claim 1, characterized by the fact that the clock (8) is formed by means of an oscillator made up of logical inverters and a capacitance resistance, fed by the energy source through the intermediary of a decoupling condenser.

FIG.1

FIG.2

FIG.3

FIG.4A

FIG.4B

FIG.5A

FIG.5B

FIG.5C